# EUROPEAN PATENT SPECIFICATION

(11) **EP 2 049 461 B1**
(45) Date of publication and mention of the grant of the patent: **21.09.2011**
(21) Application number: 07735841.4
(22) Date of filing: 10.05.2007
(51) Int. Cl.: C07C 67/08, C07C 67/03, C07C 69/24, C07C 69/52, C11C 3/10, C11C 3/06, C10L 1/02

(54) **PROCESS FOR THE PRODUCTION OF BIODIESEL**
VERFAHREN ZUR HERSTELLUNG VON BIODIESEL
PROCÉDÉ DE FABRICATION DE BIODIESEL

(30) Priority: 10.07.2006 EP 06116898
(43) Date of publication of application: 22.04.2009
(73) Proprietor: A.& A. F.lli Parodi S.r.l., 16014 Campomorone (GE) (IT); DP Lubrificanti srl, 09030 Elmas (CA) (IT)
(72) Inventor: PARODI, Augusto, I-16167 Genova (IT); MARINI, Leandro, I-16014 Ceranesi (IT)
(74) Representative: Gervasi, Gemma
(86) International application number: PCT/IB2007/051763
(87) International publication number: WO 2008/007231

(56) References cited:
- EP-A1- 1 061 120
- WO-A-2005/019153
- DE-C1- 19 620 523
- US-A- 5 424 466
- US-A1- 2002 013 486
- JAN CVENGROS, ANNA PAVLOVICOVA, GABRIELA GLADISOVA, JIRI CERNY: "Rapeseed oil methyl esters with low phosphorus content" FETT - LIPID., vol. 101, no. 7, July 1999 (1999-07), pages 261-265, XP002459290 DEWILEY-VCH VERLAG,WEINHEIM.
- BIODIESEL ASSOCIATION OF CANADA - ASSOCIATION CANADIENNE DE BIODIESEL: BIODIESEL BASICS, 2005, pages 1-4,
- BIOFUEL SYSTEMS GROUP LTD: 'Biodiesel Standards', [Online] Retrieved from the Internet: <URL:www.biofuelsystems.com/specification.h tm> [retrieved on 2010-04-30]
- BIODIESEL ASSOCIATION OF CANADA - ASSOCIATION CANADIENNE DE BIODIESEL: BIODIESEL BASICS, 2005, pages 1-4,
- BIOFUEL SYSTEMS GROUP LTD: 'Biodiesel Standards' INTERNET CITATION, [Online] Retrieved from the Internet: <URL:www.biofuelsystems.com/specification.h tm> [retrieved on 2010-04-30]

## Description

### Field of the invention

The present invention concerns a process for the production of biodiesel from triglycerides of fatty acids obtained by means of esterification with glycerol.

### State of the art

Biodiesel is a liquid fuel with a base of regenerable raw materials, such as vegetable oils or animal fats. To produce biodiesel, the molecules of long-chain triglycerides of vegetable oils and animal fats are normally decomposed by means of methanol and a catalyst, obtaining glycerine and simple ester. This chemical reaction has been known for a long time: during the Second World War fuel was already produced from rapeseed oil. However, only in recent years has the chemical transformation process been considerably improved. The by-products obtained are only glycerine and fertiliser which, when suitably purified, are equally marketable. The residual auxiliary substances, including the production water, are recycled.

In practice, all vegetable oils and fats, whether they be original or waste (for example waste food oil and fat) and all animal fats are suitable for the production of biodiesel. In particular, vegetable oils that are produced in large quantities, such as rapeseed oil, are used all over the world in numerous biodiesel systems. It has been estimated that in 1999 about 90 biodiesel systems were operating all over the world, producing about 1.3 billion litres.

Document WO2005/019153 describes e.g. a method for making alkylesters such as biofuels, from an oil source.

The publication "Rapseed oil methyl esters with low phosphorous content" , from Fett-Lipid, vol101 , no.7, july 1999, pages 261-265 describes how to lower the phosphorous content in rapeseed oil methyl esters used as an alternative diesel engine fuel.

### The main advantages of biodiesel are the following:

- it has a very low sulphur content (< 0.001%) and so it does not contribute to the phenomenon of acid rains,
- it reduces dust emissions up to 50% and is compatible with the catalyst,
- it does not contain benzol or other cancerogenous components, having a high flash point (>1 100°C),
- it is not classified as a dangerous material and so it is easy and safe to handle,
- it is highly biodegradable (99.6% after 21 days) and does not pollute the soil or the water if accidentally spilled,
- it has a high lubricating power and decreases engine wear,
- it has a closed CO₂ cycle and so its combustion in the engine produces an emission of CO₂ in the same quantity as that absorbed by plants from the air in their growth process.

Biodiesel is the only fuel that does not contribute to the increase of CO₂ in the atmosphere and therefore does not accelerate the greenhouse effect. The biodiesel molecules have very short chains in comparison with traditional diesel fuel, and this factor has a positive influence on the combustion process. Many engine experts therefore consider biodiesel an excellent fuel for self-igniting engines. For this reason too, most vehicle manufacturers (cars, trucks, including the manufacturers of diesel engines) have granted permission to use biodiesel in their products. Various national and international standards govern the quality requirements for biodiesel, establishing the limits of the residues present in the fuel, such as water or glycerine, and in this way the provisions for vehicle manufacturers are satisfied

Biodiesel may be mixed with traditional diesel fuel, in any percentage, improving its performance. In winter, as with traditional diesel fuel, it is enriched with additives, so that the driving conditions remain absolutely unchanged in sub-zero temperatures; moreover, biodiesel can be kept without problems for a period of at least 12 months.

The emission values of an engine fed with biodiesel are reduced in comparison with one running on traditional diesel fuel, as indicated in the table below:

| | |
|---|---|
| SOx | 100% less |
| CO | 5/10% less |
| HC | 20/40% less |
| Dust | 40 / 50% less |
| CO₂ | since growing plants have absorbed C02, the cycle breaks even |
| NOₓ | specific for each engine - the reduction may be as much as 10% |

The use of biodiesel in the engine does not cause a fall in performance with respect to that obtained with traditional diesel fuel.

Biodiesel may be practically obtained from all vegetable fats, from rapeseed oil, sunflower oil and soybean oil to palm oil and olive oil; even exotic oils such as Jatropha walnut oil from Nicaragua or Itolio from the berries of the Guang-Pi tree may be used. If we consider rapeseed oil, in central Europe, 1.2t of oil may be obtained from one hectare of land and transformed entirely into biodiesel. As a by-product of grinding the rapeseed, about 2t of fodder with a high protein value can be obtained, while the esterification of the oil yields 100 kg of glycerine and about 30 kg of potassic fertiliser. Biodiesel may also be produced using waste food oils or fats; from 1t of these almost 1t of biodiesel can be obtained, depending on the quantity of waste present in the raw material. Most waste food fat comes from frying, mostly from restaurants and hotels. This waste is mostly already collected and reused by fodder factories.

Instead the waste fat and oil produced in the home are unused and are usually disposed of in the drains. This causes pollution of the waste water system. Experts reckon that in central Europe an average of about 5 kg of waste food fat or oil per person per year could be obtained depending on the level of public awareness of collection, thus satisfying 1.5 to 2% of the yearly need for diesel fuel.

There are three basic ways for producing biodiesel from biolipids (fats and oils of biological origin):
1) base-catalysed transesterification of the biolipid;
2) direct acid-catalysed transesterification of the biolipid;
3) conversion of the biolipid first into its own fatty acids and then into biodiesel.

Nearly all biodiesel is currently produced by base-catalysed transesterification, as described for example in the patent applications WO2006/043281 and US2003/0167681. The good yield of these processes requires the oil to have specific properties, that is less than 1% (in weight) of suspended particles, and particles smaller than 5µm. For this reason, in the current methods for producing biodiesel, the greatest technological complications are due to the fact that the following steps are necessary:
- Filtration to 5 µm;
- Washing with warm water (liquid-liquid separation);
- Decanting;
- Heating of the oil;
- Second decanting;
- Absence of water;

For this, after the second decanting, the final stage of preparation of the oil is carried out (drying).

The transesterification reaction for producing biodiesel may be summed up as follows: 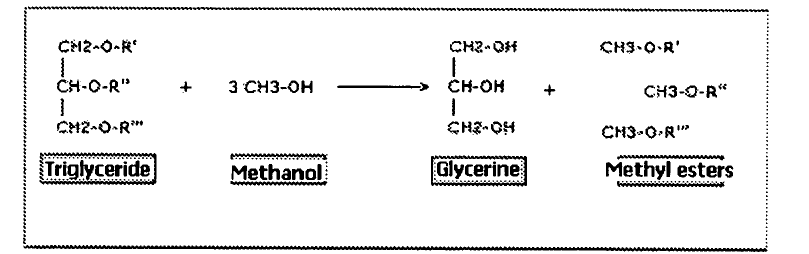 (-R', -R", R'" represent 3 different molecules of long-chain fatty acids)

During the transesterification process the trygliceride reacts with the alcohol in the presence of a catalyst, which is usually a strong base such as NaOH, or KOH.

The alcohol reacts with the triglycerides to form the monoalkyl ester (or biodiesel), releasing raw glycerol. The reaction between the biolipid (fat or oil) and the alcohol is reversible, so the alcohol must be added in excess to push the reaction towards the right and ensure complete conversion.

Before the process, particular care must be taken to monitor the quantity of water and of free fatty acids in the initial biolipid. If the level of free fatty acids and/or of humidity is too high, problems may occur with the production of soap (saponification) and the consequent partial or total inactivation of the catalyst. The catalyst is dissolved in the alcohol and the alcohol/catalyst mixture is then loaded into a closed container in which the biolipid (oil or vegetable or animal fats) is added). From then on, the system is totally closed to avoid the loss of alcohol by evaporation.

The mixture is kept stirring just above the boiling point of the alcohol, to speed up the reaction, even though in some systems it is recommended instead to perform the transformation at environment temperature. The recommended reaction time varies between 1 and 8 hours. The excess alcohol is normally used to ensure the complete conversion of the fat or oil into its esters.

The glycerine fraction thus obtained is much denser than the biodiesel fraction, and they can therefore be separated by gravity, allowing the glycerine to settle at the base of the container. In other cases a centrifuge may be used to separate the two phases more rapidly.

Once the glycerine and biodiesel phases have been separated, the excess alcohol in each phase is removed by distillation or partial evaporation. In other systems the alcohol is removed and evaporated before separating the glycerine from the esters. As a secondary product, the separated glycerine still contains unused soaps and catalysts which can be neutralised with an acid and stored as raw glycerine (water and alcohol are removed later, mainly by evaporation, to produce 80-88% pure glycerine).

Once the biodiesel has been separated from the glycerine it is purified by "washing" it in water (liquid-liquid extraction) to remove residual catalysts or soaps, then it is dried and stored.

### Description of the invention

This invention concerns the realisation of a simple and convenient method for producing biodiesel which gives a good yield and which at the same time uses raw materials of plant and/or animal origin.

How to achieve this aim is specified in claim 1, concerning a process for producing biodiesel from triglycerides obtained by means of esterification with glycerol.

The present invention therefore describes a method for the production of biodiesel according to the regulation UNI EN 14214, from by-products of the chemical and physical refining of vegetable and/or animal oils, fats and by-products of the refining of glycerine from biodiesel, fatty acids of distillation and non-distillation, hydrolytically cleaved fatty substances and distilled and non-distilled fatty acids, resulting from the cleaving of soaps, for use as biodiesel.

This process comprises the following phases:
A) direct esterification with glycerol of fatty acid substances with neutralisation number between 2 and 300,
B) neutralisation with alkali of the organic and inorganic acids resulting from phase (A)
C) transesterification reaction with alcohols of the products obtained from phase (B)
D) distillation of the esters obtained from phase (C) obtaining biodiesel according to regulations, where said distillation takes place by means of a thin layer or molecular evaporator, with absolute distillation pressure between 0 and 4 bar and a distillation temperature between 150 and 200°C.

The idea behind this invention derives from the following considerations. Biodiesel, generally defined, is a methyl ester obtained from vegetable oil, animal oil and their fats by transesterification with basic catalysts (sodium hydroxide, potassium hydroxide, sodium methylate, potassium methylate, sodium carbonate, calcium hydrate) with the use of 10-20% methanol as an alcohol to replace glycerol. The excess alcohol is generally used to put the reaction in favourable conditions for transesterification.

The vegetable oils are generally obtained by extraction from seeds, with a solvent or pressure; the animal fats are obtained by hot straining in autoclaves or with a solvent. These fatty substances normally contain free fatty acids, sterols, phospholipids, water, odorous substances and other impurities. The refining of the fatty substances involves the complete removal of all the impurities including the free fatty acids to make them usable in the production of biodiesel, in the food industry and in industry in general.

However, the refined oils still contain small quantities of free fatty acids. With alkaline catalysis these small acid fractions are transformed into soaps, giving rise to significant quantities of fatty acids combined with sodium and with potassium, all soluble in glycerine.

The presence of free fatty acids is therefore poorly tolerated in the transesterification reaction system, to the extent that it seriously conditions the result; the presence of water is equally harmful to the system, and the combination of the two negative factors is exponential. The fatty substances obtained from chemical and physical deacidification in the refining phase, understood as a by-product, and those obtained from the cleaving of soaps in the glycerine refining process, understood as a by-product, may have a neutralisation number (nn) between 2 and 300.

From this derives the intention to develop a process for the transesterification of fatty acids with a neutralisation number between 2 and 300 after direct esterification with glycerol between nn 0 and 30 followed by a further reaction with alcohols such as methanol, ethanol, propanol and butanol, with basic catalysts (such as sodium hydroxide, potassium hydroxide, sodium methylate, potassium methylate, sodium carbonate and potassium carbonate, calcium hydrate) also supported on resins, zeolites and similar or in the total absence of catalysts, with temperatures between 30 and 300°C and with absolute pressures between 1 and 100 bar to obtain biodiesel.

### Detailed description of the present invention

An acid fatty substance resulting from chemical and/or physical deacidification of oils and/or fats or a mixture of the two, or resulting from the cleaving of the soaps present in the glycerines from biodiesel, or resulting from the distillation of the fatty substances derived from chemical or physical deacidification or a mixture of the 3 above components in any percentage, with nn between 2 and 300, is placed in a reactor with a quantified weight and glycerol is added in the quantity between 0.7 and 20 mole of the necessary stoichiometric value calculated from the neutralisation number of the fatty substance used and made to react to obtain glyceric ester with nn between 0 and 30. Esterification is direct with the development of water and subsequent condensation; the concentration of the catalyst used is between 0.01 and 5% in weight of the total of the substances used.

The catalysts may be acids or salts, solids or liquids such as tin and its salts, zinc and its salts, titanium and its salts and esters or sulphuric acid, p-toluensulphonic acid, methanesulphonic acid, phosphoric acid also supported on resins, zeolites and similar. The present process also allows operation in the total absence of catalysts. The temperatures are between 50 and 300°C, while the pressure is between 0 and 10 bar absolute. The ester thus obtained is then cooled and any presence of inorganic and organic acids is neutralised with weak alkali Na₂CO₃, K₂CO₃, and others. The further transesterification reaction takes place with alcohols such as methanol, ethanol, propanol and butanol, with basic catalysts (such as sodium hydroxide, potassium hydroxide, sodium methylate, potassium methylate, sodium carbonate and potassium carbonate, calcium hydrate) also supported on resins, zeolites and similar, or in the total absence of catalysts, with temperatures between 30 and 300°C and with absolute pressures between 1 and 100 bar to obtain esters. The subsequent distillation of the ester thus obtained takes place by means of a thin layer or molecular evaporator with ester distillation feeding temperatures between 0 and +200°C with absolute distillation pressures between 0 and 20 mbar with distillation temperatures between 150 and 200°C in order to obtain biodiesel according to regulations.

The fact that distillation takes place only according to the two techniques indicated above cab be explained as follows:

Distillation (physical separation of molecules by providing heat and defined pressure conditions) is the phase in which the methyl ester previously obtained (phase C) is separated from other molecules with a higher molecular weight. The latter, in particular, are the result of oxidative and polymerisation processes due to the use of lipids, especially in processes such as frying. During distillation, when these molecules are thermally stressed and beyond a certain time limit, they undergo thermal shock with a cracking effect, releasing newly formed molecules. There are therefore new molecules which cause considerable problems since they are molecules with a low weight which distil together with the methyl ester and consequently reduce its purity.

For this reason methyl ester distilled in non optimum conditions cannot obtain a degree of purity such as to be classified as "biodiesel" according to UNI EN 14214 specifications, for which biodiesel must possess the following requirements:

| **Characteristic** | **Unit of measure** | **Values** | |
|---|---|---|---|
| | | **Min** | **Max** |
| Ester content | % (m/m) | 96.5 | |
| Density at 15°C | kg/m^3 | 860 | 900 |
| Viscosity at 40°C | mm^2/s | 3.50 | 5.00 |
| Flash point | °C 120 | | |
| Sulphur | mg/kg | | 10.0 |
| residue | % (m/m) | | 0.30 |
| Cetane number | | 51.0 | |
| Sulphurated ash | % (m/m) 0.02 | | |
| Water content | mg/kg | | 500 |
| Total contamination | mg/kg | | 24 |
| Corrosion on copper | | Class 1 | |
| Oxidation stability, 110°C | h (hours) | 6.0 | |
| Acidity | mg KOH/g 0.5 | | |
| Iodine number | gr 12/100gr | | 120 |
| Linolenic acid methyl ester | % (m/m) | 1 | 2.0 |
| Polyunsaturated methyl esters >=4 double bonds | % (m/m) | | 1 |
| Methanol | % (m/m) | | 0.20 |
| Monoglycerides | % (m/m) | | 0.80 |
| Diglycerides | % (m/m) | | 0.20 |
| Triglycerides | % (m/m) | | 0.20 |
| Free glycerol | % (m/m) | | 6.02 |
| Total glycerol | % (m/m) 0.25 | | |
| Metals group I (Na+K) | mg/kg | | 5.0 |
| Metals group II (Ca+Mg) | mg/kg | | 5.0 |
| Phosphor | mg/kg | | 10.0 |

On the basis of these specifications and particularly of the high-acidity methyl ester content, it has been proved in the laboratory and also in industrial systems that it is exclusively with distillation technologies that make use of a thin-layer of molecular evaporator that it is possible to obtain a product defined biodiesel.

The advantages of the present invention are due to the fact that this process:
- contemplates the esterification of the acidity present in the fatty substances by means of glycerine with the exclusion of methanol, ethanol, propanol, butanol and other alcohols;
- excludes rectifying to eliminate condensation water due to the combination of alcohol and acid in the synthesis process;
- does not require purifications of the initial fatty substance;
- allows considerable energy saving.

Moreover, classic chemistry contemplates the direct reaction between methanol and fatty acid for the production of biodiesel. However, as biodiesel is a mass product it is important to check the costs of this direct reaction, since high production costs would prevent the use of these by-products. In fact, high excesses of methanol are necessary in order to arrive at low acidity with the direct reaction of fatty acid + methanol. The methanol forms a mixture with the acid-alcohol reaction water which can be separated only with a rectifying column

The following costs to complete the reaction have been calculated:
- cost of the acid/alcohol reaction (gr/gr)
- rectification of the H₂O/alcohol mixture
- reactor dimensions (since we are working with strong excesses of alcohol about 5 times greater than stoichiometry, the dimensions of the reactor are calculated accordingly)
- rectification of the excess methanol used to complete the reaction
- reaction times
- volume of cooling H2O
- investment costs

From the calculations made, the costs amount to about 0.23euro/kg against 0.02 euro/kg for the reaction with glycerine. This substantial difference in fact allows an industrial use of the present process, which does not occur for the classic technique.

### Example 1

1000gr of acid fatty substance resulting from the refining of rapeseed oil with an acidity of 120 mg KOH/gr, 66gr of glycerol, 4.26gr of 2-ethylhexyl titanate are placed in a stirred reactor SS316 and heated. The reaction begins at 130°C and ends at 230°C in 8 hours. The absolute pressure up to the sixth hour is 1 bar, in the last 2 hours the absolute pressure is residual 5-10mbar, the final acidity of the triglyceride is 0.5mg KOH/gr. The temperature is brought to 30°C, the pressure is brought to one bar absolute and any mineral and organic acids present are neutralised with 4,634 mg of Na₂CO₃ on gr. of product, followed by dehydration of the mass by means of a vacuum; 113gr of methanol and 4.55gr of sodium methylate are introduced into the system. The methyl ester that forms is deprived of glycerine and of the residual methanol. The methyl ester is distilled to obtain biodiesel exclusively by means of the process that uses a thin layer or molecular evaporator with ester distillation feeding temperatures between 0 and +200°C with absolute distillation pressures between 0 and 4 bar and with distillation temperatures between 150 and 200°C in order to obtain biodiesel according to regulations.

### Example 2

1000gr of acid fatty substance resulting from the refining of sunflower oil with an acidity of 120 mg KOH/gr, 66gr of glycerol, 4.26gr of 2-ethylhexyl titanate are placed in a stirred reactor SS316 and heated. The reaction begins at 130°C and ends at 230°C in 8 hours. The absolute pressure up to the sixth hour is 1 bar, in the last 2 hours the absolute pressure is residual 5-10 mbar, the final acidity of the triglyceride is 0.5mg KOH/gr. The temperature is brought to 30°C, the pressure is brought to one bar absolute, the mineral and organic acids are neutralised with 4,634 mg of Na₂CO₃ on gr. of product, followed by dehydration of the mass by means of a vacuum; 113gr of methanol and 4.55gr of sodium methylate are introduced into the system. The methyl ester that forms is deprived of glycerine and of the residual methanol. The methyl ester is then distilled to obtain biodiesel exclusively by means of the process that uses a thin layer or molecular evaporator with ester distillation feeding temperatures between 0 and +200°C with absolute distillation pressures between 0 and 4 bar and with distillation temperatures between 150 and 200°C in order to obtain biodiesel according to regulations.

### Example 3

1000gr of acid fatty substance resulting from the refining of almond oil with an acidity of 120 mg KOH/gr, 66gr of glycerol, 4.26gr of 2-ethylhexyl titanate are placed in a stirred reactor SS316 and heated. The reaction begins at 130°C and ends at 230°C in 8 hours. The absolute pressure up to the sixth hour is 1 bar, in the last 2 hours the absolute pressure is residual 5-10mbar, the final acidity of the triglyceride is 0.5 mg KOH/gr. The temperature is brought to 30°C, the pressure is brought to one bar absolute and the mineral and organic acids are neutralised with 4,634 mg of Na₂CO₃ on gr. of product, followed by dehydration of the mass by means of a vacuum; 113gr of methanol and 4.55gr of sodium methylate are introduced into the system. The methyl ester that forms is deprived of glycerine and of the residual methanol. The methyl ester is distilled to obtain biodiesel exclusively by means of the process that uses a thin layer or molecular evaporator with ester distillation feeding temperatures between -10 and +300°C with absolute distillation pressures between 0 and 1 bar and with distillation temperatures between 80 and 300°C in order to obtain biodiesel according to regulations.

### Example 4

1000gr of acid fatty substance resulting from the refining of biodiesel glycerol with an acidity of 40 mgKOH/gr, 30gr of glycerol, 1.03gr of tin chloride are placed in a stirred reactor SS316 . The reaction begins at 130°C and ends at 230°C in 8 hours at absolute pressure. The absolute pressure up to the sixth hour is 1 bar, in the last 2 hours the absolute pressure is residual 1-5mbar, the final acidity of the triglyceride is 0.1 mg KOH/gr.

The temperature is brought to 30°C, the pressure is brought to one bar absolute and the mineral and organic acids are neutralised with 4,634 mg of Na₂CO₃ on gr. of product, followed by dehydration of the mass by means of a vacuum; 183gr of methanol and 4.00gr of sodium methylate are introduced into the system. The methyl ester that forms is deprived of glycerine and of the residual methanol. The methyl ester is distilled to obtain biodiesel exclusively by means of the process that uses a thin layer or molecular evaporator with ester distillation feeding temperatures between 0 and +200°C with absolute distillation pressures between 0 and 4 bar and with distillation temperatures between 150 and 200°C in order to obtain biodiesel according to regulations.

Comparative examples of distillation methods:

### 1) CONTINUOUS THIN FILM DISTILLER

Exchange surface 16 m²
No. revs 200 rpm

### OPERATIVE CONDITIONS

| | |
|---|---|
| Methyl ester feed | 2000-4000 1/h |
| Methyl ester purity | 88% |
| Acidity | 0,4 mg of KOH/gr |
| Evaporator project pressure | 1 mbar abs |
| Heating fluid temperature | 260 °C |

| **FEED** | **VACUUM** | **DISTILLATE** | **METHYL ESTER IN THE DISTILLATE** | **DISTILLATION RESIDUE** | **METHYL ESTER IN THE RESIDUE** | **ACIDITY** |
|---|---|---|---|---|---|---|
| ***l*/*h*** | ***mbar*** | ***%*** | ***%*** | ***%*** | ***%*** | ***mg*/*gr KOH*** |
| 2000 | 6 | 90 | 98 | 10 | 5 | 0,4 |
| 3000 | 8 | 90 | 98 | 10 | 7 | 0,4 |
| 4000 | 9 | 89 | 99 | 11 | 11 | 0,3 |

The **CONTINUOUS THIN FILM** distillation process allows biodiesel according to regulations to be obtained according to UNI EN 14214 specifications and in particular as regards methyl ester content and acidity.

### 2) CONTINUOUS MOLECULAR DISTILLER (SHORT PATH)

Exchange surface 3 m²

| | |
|---|---|
| No. revs | 200 rpm |
| OPERATIVE CONDITIONS | |
| Methyl ester feed | 400-600 l/h |
| Methyl ester purity | 92% |
| Acidity | 0.3 mg of KOH/gr |
| Evaporator project pressure | 0,5-1 mbar abs |
| Heating fluid temperature | 220°C |

| **FEED** | **VACUUM** | **DISTILLATE** | **METHYL ESTER IN THE DISTILLATE** | **DISTILLATION RESIDUE** | **METHYL ESTER IN THE RESIDUE** | **ACIDITY** |
|---|---|---|---|---|---|---|
| ***l*/*h*** | ***mbar*** | ***%*** | ***%*** | ***%*** | ***%*** | ***KOH*/*gr*** |
| 400 | 0,5 | 94 | 98 | 6 | 1 | 0,3 |
| 500 | 0,5 | 94 | 98 | 6 | 3 | 0,3 |
| 600 | 1 | 93 | 99 | 7 | 3 | 0,2 |

The **CONTINUOUS SHORT PATH** distillation process allows biodiesel according to regulations to be obtained according to UNI EN 14214 specifications and in particular as regards methyl ester content and acidity.

### 3) CONTINUOUS FALLING FILM DISTILLER

| | |
|---|---|
| Exchange surface | 30 m² |
| OPERATIVE CONDITIONS | |
| Methyl ester feed | 3000-5000 l/h |
| Methyl ester purity | 88% |
| Acidity | 0.4 mg of KOH/gr |
| Evaporator project pressure | 1 mbar abs |
| Heating fluid temperature | 260 °C |

| **FEED** | **VACUUM** | **DISTILLATE** | **METHYL ESTER IN THE DISTILLATE** | **DISTILLATION RESIDUE** | **METHYL ESTER IN THE RESIDUE** | **ACIDITY** |
|---|---|---|---|---|---|---|
| ***l*/*h*** | ***mbar*** | ***%*** | ***%*** | ***%*** | ***%*** | ***KOH*/*gr*** |
| 3000 | 60 | 98 | 90 | 2 | - | 2,2 |
| 4000 | 55 | 98 | 90 | 2 | 1 | 1,8 |
| 5000 | 55 | 96 | 91 | 3 | 1 | 1,7 |

The **CONTINUOUS FALLING FILM** distillation process does not allow biodiesel according to regulations to be obtained according to UNI EN 14214 specifications and in particular as regards methyl ester content and acidity.

### 4) CONTINUOUS FLASH DISTILLER

| | |
|---|---|
| Exchange surface | 30 m² |
| OPERATIVE CONDITIONS | |
| Methyl ester feed | 3000-5000 l/h |
| Methyl ester purity | 92 % |
| Acidity | 0.3 mg of KOH/gr |
| Evaporator project pressure | 1 mbar abs |

| **FEED** | **VACUUM** | **DISTILLATE** | **METHYL ESTER IN THE DISTILLATE** | **DISTILLATION RESIDUE** | **METHYL ESTER IN THE RESIDUE** | **ACIDITY** |
|---|---|---|---|---|---|---|
| ***l*/*h*** | ***mbar*** | ***%*** | ***%*** | ***%*** | ***%*** | ***KOH*/*gr*** |
| 3000 | 70 | 97 | 92 | 3 | 3 | 2 |
| 4000 | 65 | 96,5 | 92,5 | 3,5 | 3 | 2 |
| 5000 | 65 | 96 | 93 | 4 | 3 | 1,8 |

The **CONTINUOUS FLASH** distillation process does not allow biodiesel according to regulations to be obtained according to UNI EN 14214 specifications and in particular as regards methyl ester content and acidity.

## Claims

1. Method for the production of biodiesel according to the regulation UNI EN 14214, from by-products of the chemical and physical refining of vegetable and/or animal oils, fats and by-products of the refining of glycerine from biodiesel, fatty acids of distillation and non-distillation, hydrolytically cleaved fatty substances and distilled and non-distilled fatty acids, resulting from the cleaving of soaps, for use as biodiesel comprising the following phases:
A) direct esterification with glycerol of fatty acid substances with neutralisation number between 2 and 300 to obtain glyceric esters with nn between 0 and 30, wherein glycerol is present in a quantity between 0.7 and 20mole of the necessary stoichiometric value.;
B) neutralisation with alkali of the organic and inorganic acids resulting from phase (A)
C) transesterification reaction with alcohols of the products obtained from phase (B)
D) distillation of the esters obtained from phase (C) where said distillation takes place by means of a thin layer or molecular evaporator wherein the ester distillation feeding temperature is between 0 and +200°C, the absolute distillation pressure is between 0 and 4 bar and the distillation temperature is between 150 and 200°C.

2. Method according to claim 1,
wherein the catalysts used are acids or salts, solids or liquids such as tin and its salts, zinc and its salts, titanium and its salts and esters or sulphuric acid, p-toluensulphonic acid, methanesulphonic acid or phosphoric acid, optionally supported on resins, zeolites and similar.

3. Method according to claim 2,
wherein the concentration of the catalyst used is between 0.01 and 5% in weight of the total of the substances used.

4. Method according to one of the claims from 1 to 3,
wherein in phase (A) the pressure is between 0 and 10 bar absolute.

5. Method according to one of the claims from 1 to 4,
where the reaction (A) takes place without a catalyst.

6. Method according to one of the previous claims,
wherein the transesterification reaction (C) takes place with alcohols such as methanol, ethanol, propanol and butanol, with the use of basic catalysts, such as sodium hydroxide, potassium hydroxide, sodium methylate, potassium methylate, sodium carbonate, calcium hydrate.

7. Method according to claim 6,
wherein the reaction takes place at a temperature between 30 and 300°C with absolute pressure between 1 and 100 bar.

## Patentansprüche

1. Verfahren für die Herstellung von Biodiesel in Übereinstimmung mit der Vorschrift UNI EN 14214 aus Nebenprodukten der chemischen und physikalischen Raffination pflanzlicher und/oder tierischer Öle, Fette und aus Nebenprodukten der Raffination von Glycerin aus Biodiesel, Fettsäuren, der Destillation und Nichtdestillation hydrolytisch gespaltener Fettsubstanzen und destillierter und nichtdestillierter Fettsäuren, die sich aus der Spaltung von Seifen ergeben, zur Verwendung als Biodiesel, wobei das Verfahren die folgenden Phasen umfasst:
A) direkte Veresterung mit Glycerin von Fettsäuresubstanzen mit der Neutralisationszahl zwischen 2 und 300 zum Erhalten von Glycerinestern mit einer nn zwischen 0 und 30, wobei Glycerin in einer Menge zwischen 0,7 und 20 Mol des notwendigen stöchiometrischen Werts vorhanden ist;
B) Neutralisation mit Alkali der organischen und anorganischen Säuren, die sich aus Phase (A) ergeben,
C) Umesterungsreaktion mit Alkoholen der aus Phase (B) erhaltenen Produkte,
D) Destillation der aus Phase (C) erhaltenen Ester, wobei die genannte Destillation mittels eines Dünnschicht- oder Molekularverdampfers stattfindet, wobei die Esterdestillations-Zufuhrtemperatur zwischen 0 und +200 °C liegt, der Destillationsabsolutdruck zwischen 0 und 4 Bar liegt und die Destillationstemperatur zwischen 150 und 200°C liegt.

2. Verfahren gemäß Anspruch 1,
wobei die verwendeten Katalysatoren Säuren oder Salze, Festkörper oder Flüssigkeiten wie etwa Zinn und seine Salze, Zink und seine Salze, Titan und seine Salze und Ester oder Schwefelsäure, p-Toluolsulfonsäure, Methansulfonsäure oder Phosphorsäure, optional gehalten an Harzen, Zeolithen und Ähnlichem, sind.

3. Verfahren gemäß Anspruch 2,
wobei die Konzentration des verwendeten Katalysators zwischen 0,01 und 5 Gew.-% der insgesamt verwendeten Substanzen liegt.

4. Verfahren gemäß einem der Ansprüche 1 bis 3,
wobei der Druck in Phase (A) zwischen 0 und 10 Bar absolut beträgt.

5. Verfahren gemäß einem der Ansprüche 1 bis 4,
bei dem die Reaktion (A) ohne einen Katalysator stattfindet.

6. Verfahren gemäß einem der vorherigen Ansprüche,
bei dem die Umesterungsreaktion (C) mit Alkoholen wie etwa Methanol, Ethanol, Propanol und Butanol, unter Verwendung basischer Katalysatoren wie etwa Natriumhydroxid, Kaliumhydroxid, Natriummethylat, Kaliummethylat, Natriumcarbonat, Calciumhydrat stattfindet.

7. Verfahren gemäß Anspruch 6,
bei dem die Reaktion bei einer Temperatur zwischen 30 und 300 °C bei einem Absolutdruck zwischen 1 und 100 Bar stattfindet.

## Revendications

1. Procédé de production de biodiesel conformément à la réglementation UNI EN 14214, à partir de sous-produits de l'affinage chimique et physique d'huiles, de graisses végétales et/ou animales et de sous-produits de l'affinage de glycérine à partir de biodiesel, d'acides gras de distillation et de non distillation, de substances grasses clivées par voie hydrolytique et d'acides gras distillés et non distillés, résultant du clivage de savons, pour utilisation en tant que biodiesel, comportant les phases suivantes :
A) estérification directe à l'aide de glycérol des substances d'acides gras avec un indice de neutralisation compris entre 2 et 300 pour obtenir des esters glycériques avec nn compris entre 0 et 30, sachant que le glycérol est présent dans une quantité comprise entre 0,7 et 20 moles de la valeur stoechiométrique nécessaire ;
B) neutralisation avec des alcalins des acides organiques et minéraux résultant de la phase (A)
C) réaction de transestérification avec des alcools des produits obtenus à partir de la phase (B)
D) distillation des esters obtenus partir de la phase (C) où ladite distillation a lieu au moyen d'un évaporateur à couches mince ou d'un évaporateur moléculaire, la température d'alimentation de distillation des esters étant comprise entre 0 et 200°C, la pression absolue de distillation étant comprise entre 0 et 4 bar et la température de distillation étant comprise entre 150 et 200 °C.

2. Procédé selon la revendication 1, selon lequel que les catalyseurs utilisés sont des acides ou des sels, des solides ou des liquides tels que l'étain et ses sels, le zinc et ses sels, le titane et ses sels, et des esters ou l'acide sulfurique, l'acide p-toluènesulfonique, l'acide méthanesulfonique ou l'acide phosphorique, fixés en option sur des résines, des zéolites et similaires.

3. Procédé selon la revendication 2, selon lequel que la concentration du catalyseur utilisé est compris entre 0,01 et 5 % en poids du total des substances utilisées.

4. Procédé selon l'une quelconque des revendications 1 à 3, selon lequel que dans la phase (A) la pression est comprise entre 0 et 10 bar absolus.

5. Procédé selon l'une quelconque des revendications 1 à 4, selon lequel que la réaction (A) a lieu sans catalyseur.

6. Procédé selon l'une quelconque des revendications précédentes, selon lequel que la réaction de transestérification (C) a lieu avec des alcools tels que le méthanol, l'éthanol, le propanol, et le butanol, avec l'utilisation de catalyseurs basiques, comme l'hydroxyde de sodium, l'hydroxyde de potassium, le méthylate de sodium, le méthylate de potassium, le carbonate de sodium, l'hydrate de calcium.

7. Procédé selon la revendication 6, selon lequel que la réaction a lieu à une température comprise entre 30 et 300 °C avec une pression absolue comprise entre 1 et 100 bar.
